# EUROPEAN PATENT APPLICATION

(11) **EP 4 620 393 A1**
(43) Date of publication of application: **24.09.2025**
(21) Application number: 24774156.4
(22) Date of filing: 20.03.2024
(51) Int. Cl.: A61B 5/1455, A61B 5/145, A61B 5/00

(54) **BLOOD GLUCOSE MONITORING METHOD, GRAPHICAL INTERFACE, AND RELATED APPARATUS**

(30) Priority: 22.03.2023 CN 202310326223
(71) Applicant: Huawei Technologies Co., Ltd., Shenzhen, Guangdong 518129 (CN); China International Exchange and Promotive Association For Medical And Health Care, Beijing 100026 (CN)
(72) Inventor: ZHOU, Hui, Shenzhen, Guangdong 518129 (CN); CHEN, Maolin, Shenzhen, Guangdong 518129 (CN); LI, Luping, Shenzhen, Guangdong 518129 (CN); JIA, Ziqian, Shenzhen, Guangdong 518129 (CN); LI, Liangzhi, Shenzhen, Guangdong 518129 (CN); ZHANG, Xuexing, Shenzhen, Guangdong 518129 (CN)
(74) Representative: Körber, Martin Hans
(86) International application number: PCT/CN2024/082751
(87) International publication number: WO 2024/193591

(57) **Abstract**

This application discloses a blood glucose monitoring method, a graphical interface, and a related apparatus. In the method, a wearable device collects a PPG signal of a user used to reflect blood glucose of the user within a time period, removes a low-quality PPG signal collected when the user is affected by an external factor, and determines, based on a reserved PPG signal, a quantity of occurrences of a blood glucose abnormality and/or a blood glucose risk of the user within the time period. It can be learned that, when data used to reflect the blood glucose of the user is collected, the user may be affected by the external factor, and the device may remove the PPG signal collected when the user is affected by the external factor. In this way, a status of the user does not need to be considered in a blood glucose monitoring process, thereby achieving an effect of unperceived blood glucose monitoring.

## Description

This application claims priority to Chinese Patent Application No. 202310326223.6, filed with the China National Intellectual Property Administration on March 22, 2023 and entitled "BLOOD GLUCOSE MONITORING METHOD, GRAPHICAL INTERFACE, AND RELATED APPARATUS", which is incorporated herein by reference in its entirety.

### TECHNICAL FIELD

This application relates to the field of terminals and communication technologies, and in particular, to a blood glucose monitoring method, a graphical interface, and a related apparatus.

### BACKGROUND

Diabetes is highly prevalent but poorly known in the population. In addition, diabetes poses significant risks and is more likely to lead to numerous serious complications, severely affecting people's quality of life. If long-term, timely, and accurate routine monitoring of blood glucose statuses of people can be achieved, and users are prompted to change their lifestyle, blood glucose of diabetes patients can be controlled in a timely and effective manner, or healthy users can be effectively prevented from becoming diabetes users through early discovery, early treatment, and early control.

It can be learned that routine monitoring of blood glucose is of great significance in improving people's quality of life and mitigating diabetes-related complications.

### SUMMARY

This application provides a blood glucose monitoring method, a graphical interface, and a related apparatus, to implement an effect of unperceived monitoring of blood glucose of a user.

According to a first aspect, an embodiment of this application provides a blood glucose monitoring method. The method is applied to a wearable electronic device, and the method includes: The electronic device collects a first photoplethysmogram PPG signal of a user within a first time period. The electronic device determines, based on a second PPG signal in the first PPG signal, a quantity of occurrences of a blood glucose abnormality and/or a blood glucose risk of the user within the first time period, where the blood glucose risk indicates a frequency at which the blood glucose abnormality occurs within the first time period. The second PPG signal does not include a PPG signal collected when the user is affected by an external factor, and the external factor includes one or more of the following: diet, medication, exercise, or psychology.

According to the method provided in the first aspect, in a process of collecting a PPG signal, a PPG signal collected when the user is affected by the external factor can be deleted, thereby ensuring accuracy of blood glucose monitoring. In addition, a collection process of data does not limit a status of the user, and the user does not need to consciously keep still, keep a stable mood, or the like. In this method, data collected when the status of the user is abnormal can be automatically removed, that is, data collected when the user is affected by the external factor is removed, so that the user perceives no blood glucose monitoring. This implements unperceived blood glucose monitoring, improves experience of the user during blood glucose monitoring, and meets a requirement of the user for routine blood glucose monitoring.

With reference to the first aspect, in some possible implementations, the method further includes: The electronic device displays the quantity of occurrences of the blood glucose abnormality and/or the blood glucose risk of the user within the first time period.

In this way, the user can view an evaluation result of the blood glucose of the user, and perform self-management and control on the blood glucose.

With reference to the first aspect, in some possible implementations, the method further includes: The electronic device displays collection progress of data, where the collection progress indicates that the user has collected data within the first time period and has not collected data within a second time period, and the second time period is different from the first time period.

In this way, the user can learn a collection status of the data, and cooperate with the device to facilitate the collection progress of the data, so that the electronic device can collect enough data.

With reference to the first aspect, in some possible implementations, the collection progress is displayed as a progress bar around an edge of a watch face, and the progress bar represents 24 hours of a day.

In this way, the collection progress may be cleverly combined with the watch face, so that the user can learn about collection progress of the PPG signal in a day.

With reference to the first aspect, in some possible implementations, an occurred exogenous event and occurrence time of the exogenous event are further marked in the progress bar, and the exogenous event includes one or more of the following: diet, medication, exercise, sleep, or wake-up.

In this way, an association between the blood glucose of the user and the exogenous event may be highlighted, so that the user, based on the exogenous event, controls a change of the blood glucose of the user by adjusting the exogenous event, thereby enhancing a self-management capability of the user on the blood glucose.

With reference to the first aspect, in some possible implementations, the quantity of occurrences of the blood glucose abnormality within the first time period is a quantity of samples with the blood glucose abnormality in a plurality of samples, and one of the samples includes a second PPG signal within preset duration within the first time period.

In this way, the electronic device may monitor the blood glucose of the user for a plurality of times, and collect statistics on and evaluate a blood glucose status of the user within the time period.

With reference to the first aspect, in some possible implementations, the blood glucose risk is equal to a ratio of the quantity of occurrences of the blood glucose abnormality within the first time period to a quantity of the plurality of samples.

With reference to the first aspect, in some possible implementations, the plurality of samples include a first sample, whether there is a blood glucose abnormality within first preset duration in which the first sample is located is identified by using a first blood glucose model based on a feature of the first sample, where the first blood glucose model is obtained through training based on a feature of a PPG signal whose blood glucose abnormality is known within a plurality of time periods.

With reference to the first aspect, in some possible implementations, the method further includes: The electronic device obtains basic information of the user, where the first blood glucose model is obtained through screening and matching from a plurality of blood glucose models based on the basic information.

It can be learned that selecting a model suitable for the user can achieve more accurate and appropriate identification of the blood glucose of the user.

With reference to the first aspect, in some possible implementations, that the electronic device determines, based on a second PPG signal in the first PPG signal, a blood glucose risk of the user within the first time period specifically includes: The electronic device extracts a feature of the second PPG signal in the first PPG signal. The electronic device determines the blood glucose risk within the first time period by using a second blood glucose model based on the feature of the second PPG signal, where the second blood glucose model is obtained through training based on a feature of a PPG signal whose blood glucose risk is known within a plurality of time periods.

With reference to the first aspect, in some possible implementations, in a process in which the electronic device collects the first PPG signal of the user within the first time period, the method further includes: When duration in which the electronic device collects a PPG signal does not reach specified duration, the electronic device outputs prompt information, to prompt the user to continue to wear the electronic device to collect the PPG signal.

In this way, the user can be reminded to perform adjustment in a timely manner, so that the electronic device collects enough PPG signals.

With reference to the first aspect, in some possible implementations, the method further includes: The electronic device displays a first risk curve, where the first risk curve indicates the blood glucose risk determined on each of a plurality of days.

In this way, the user can learn about fluctuation of the blood glucose risk within several days based on the risk curve, and review and summarize the blood glucose status of the user, thereby facilitating self-management of a change of the blood glucose of the user.

With reference to the first aspect, in some possible implementations, the method further includes: The electronic device displays a first bar chart, where the first bar chart indicates a time period in which a PPG signal has been collected and a time period in which a PPG signal has not been collected on each of the plurality of days.

In this way, the user can view distribution of collection time of the data of the user in several days, to facilitate adjustment of a lifestyle of the user in a timely manner and more effective data collection by the device.

According to a second aspect, an embodiment of this application provides an electronic device, including a memory, one or more processors, and one or more programs. When the one or more processors execute the one or more programs, the electronic device is enabled to implement the method according to any one of the first aspect or the possible implementations of the first aspect.

According to a third aspect, an embodiment of this application provides a computer-readable storage medium including instructions. When the instructions are run on an electronic device, the electronic device is enabled to perform the method according to any one of the first aspect or the possible implementations of the first aspect.

According to a fourth aspect, an embodiment of this application provides a computer program product. When the computer program product runs on a computer, the computer is enabled to perform the method according to any one of the first aspect or the possible implementations of the first aspect.

### BRIEF DESCRIPTION OF DRAWINGS

FIG. 1 is a diagram of a hardware structure of an electronic device 100 according to an embodiment of this application;
FIG. 2A to FIG. 2E show some user interfaces according to an embodiment of this application;
FIG. 3 is an overall method flowchart of a blood glucose monitoring method according to an embodiment of this application;
FIG. 4 is a diagram of a risk curve marked with reference values according to an embodiment of this application;
FIG. 5 is a diagram of risk curves of individuals of different age groups according to an embodiment of this application;
FIG. 6 is a detailed method flowchart for identifying a blood glucose abnormality event by an electronic device 100 by using a short-term blood glucose model according to an embodiment of this application;
FIG. 7 is a diagram of a curve drawn based on a PPG signal collected within a continuous time period according to an embodiment of this application;
FIG. 8 is a detailed method flowchart for determining a blood glucose risk by an electronic device 100 by using a long-term blood glucose model according to an embodiment of this application; and
FIG. 9 is a diagram of a structure of a blood glucose monitoring apparatus according to an embodiment of this application.

### DESCRIPTION OF EMBODIMENTS

The technical solutions according to embodiments of this application are clearly and completely described in the following based on the accompanying drawings. In descriptions of embodiments of this application, unless otherwise specified, "/" indicates "or". For example, A/B may indicate A or B. The term "and/or" in this specification merely describes an association relationship between associated objects, and indicates that three relationships may exist. For example, A and/or B may indicate the following three cases: Only A exists, both A and B exist, and only B exists. In addition, in the descriptions of embodiments of this application, "a plurality of" means two or more.

The following terms "first" and "second" are merely intended for a purpose of description, and shall not be understood as an indication or implication of relative importance or implicit indication of a quantity of indicated technical features. Therefore, a feature limited by "first" and "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is source code written in a specific computer language such as Java or an extensible markup language (extensible markup language, XML). Interface source code is parsed and rendered on an electronic device, and is finally presented as content that can be identified by the user. A frequently-used representation form of the user interface is a graphical user interface (graphical user interface, GUI), and is a user interface that is displayed in a graphical manner and that is related to a computer operation. The user interface may be a visual interface element such as a text, an icon, a button, a menu, a tab, a text box, a dialog box, a status bar, a navigation bar, or a Widget that is displayed on a display of the electronic device.

Diabetes is a metabolic disease characterized by high blood glucose. Long-term high blood glucose may lead to chronic damage and functional impairment of various tissues, particularly eyes, kidneys, heart, blood vessels, and nerves. It can be learned that effective diabetes management requires real-time monitoring of blood glucose concentration of patients.

Common blood glucose monitoring methods include: (1) measuring a physiological parameter related to human metabolism when a user is still, and estimating blood glucose of the user based on basic information of a human body, meal information, and the like; and (2) measuring a glucose absorption amount in a wrist blood vessel of the user by using an infrared sensing technology, to measure a blood glucose value of the user. However, before the measurement, fingertip blood of the user needs to be collected for calibration, and the user needs to keep still for a time period during the collection.

In method 1, while non-invasive blood glucose measurement is achieved, the user needs to cooperate to keep still in a measurement process. In method 2, the fingertip blood of the user needs to be collected before the blood glucose measurement, and an effect of non-invasive blood glucose monitoring cannot be achieved. It can be learned that, neither method 1 nor method 2 can achieve unperceived blood glucose detection, and the user needs to cooperate with an instrument in a blood glucose detection process to complete the blood glucose detection. Consequently, experience of the user in a blood glucose measurement process is poor.

Therefore, how to improve experience of the user during blood glucose monitoring to achieve an effect of unperceived blood glucose monitoring is an urgent problem to be resolved currently.

An embodiment of this application provides a blood glucose monitoring method. In the method, a wearable device collects a photoplethysmogram (Photoplethysmogram, PPG) signal used to reflect blood glucose of a user within a time period, deletes a PPG signal collected when the user is affected by an external factor, and determines, based on a reserved PPG signal, a quantity of occurrences of a blood glucose abnormality and/or a blood glucose risk of the user within the time period.

In the method, a PPG signal is collected through a photoelectric sensor in the wearable device. The photoelectric sensor may detect different intensities of reflected light after the reflected light is absorbed by human blood and tissue, and record a change of a blood vessel volume in a cardiac cycle. When concentration of glucose in human blood increases, absorption of light by glucose in the blood changes, resulting in a slight change of a peak value in the PPG signal. Therefore, the PPG signal may be used to detect a blood glucose status of the user, to achieve an effect of non-invasive blood glucose monitoring.

The external factor may include but is not limited to one or more of the following: diet, medication, exercise, psychology, or the like. For example, in an exercise process of the user, utilization of glucose by muscles in a body is increased, resulting in unstable fluctuation of blood glucose. For another example, when under excessive stress from work, study, or life, the user may experience negative emotions such as anxiety and irritability, which may result in autonomic nervous system dysfunction and cause abnormal elevations or decreases of blood glucose. It can be learned that the external factor affects a change of blood glucose in the user body. Consequently, the blood glucose collected under impact of the external factor is abnormal, and cannot represent a blood glucose status of the user in a general status.

Therefore, according to the blood glucose monitoring method provided in this embodiment of this application, in a process of collecting a PPG signal, a PPG signal collected when the user is affected by the external factor can be deleted, thereby ensuring accuracy of blood glucose monitoring. In addition, a collection process of data also does not limit a status of the user, and the user does not need to keep still, keep a stable mood, or the like. In this method, data collected when the status of the user is abnormal can be automatically removed, that is, data collected when the user is affected by the external factor is removed, so that the user perceives no blood glucose monitoring. This implements unperceived blood glucose monitoring, improves experience of the user during blood glucose monitoring, and meets a requirement of the user for routine blood glucose monitoring.

The blood glucose monitoring method provided in this embodiment of this application may be applied to a wearable electronic device, such as a smart band or a smartwatch, and a PPG signal used to determine blood glucose of a user is collected in real time through a photoelectric sensor in the electronic device, to implement real-time routine blood glucose monitoring for the user.

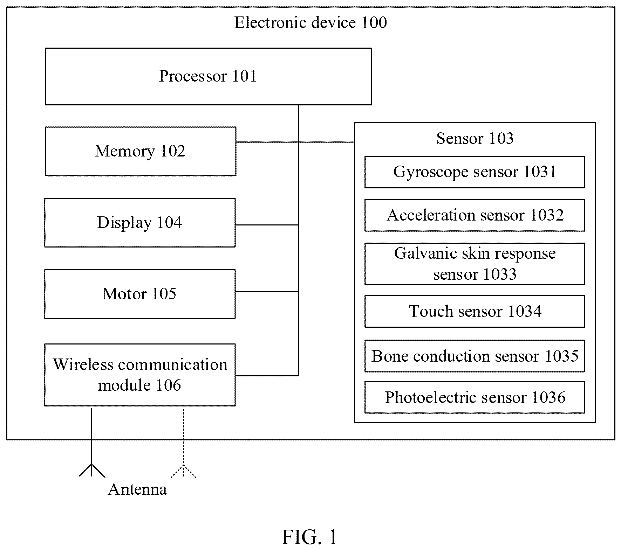

The following uses an electronic device 100 shown in FIG. 1 as an example to describe a hardware structure of the electronic device 100 provided in an embodiment of this application.

**FIG. 1** **is a diagram of a hardware structure of an electronic device 100.**

The electronic device 100 may be a wearable device such as a smart band or a smartwatch. A specific type of the electronic device 100 is not specifically limited in embodiments of this application.

The electronic device 100 may include a processor 101, a memory 102, a sensor 103, a display 104, a motor 105, a wireless communication module 106, and the like.

The processor 101 may include one or more processing units. For example, the processor 101 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on an instruction operation code and a time sequence signal, to complete control of instruction reading and instruction execution.

A memory may be further disposed in the processor 101, and is configured to store instructions and data. In some embodiments, the memory in the processor 101 is a cache. The memory may store instructions or data just used or cyclically used by the processor 101. If the processor 101 needs to use the instructions or the data again, the processor may directly invoke the instructions or the data from the memory. This avoids repeated access and reduces waiting time of the processor 101, thereby improving system efficiency.

In this embodiment of this application, the processor 101 may be configured to determine, based on the PPG signal, a quantity of occurrences of a blood glucose abnormality and/or a blood glucose risk of the user. The quantity of occurrences of the blood glucose abnormality may include: a quantity of occurrences of high blood glucose and/or a quantity of occurrences of low blood glucose. The blood glucose risk may indicate a frequency at which the high blood glucose and/or low blood glucose of the user occur/occurs. For detailed descriptions of determining the quantity of occurrences of the blood glucose abnormality and/or the blood glucose risk based on the PPG signal, refer to subsequent descriptions. Details are not described herein.

The memory 102 may include one or more random access memories (random access memories, RAMs) and one or more non-volatile memories (non-volatile memories, NVMs).

The random access memory may be directly read and written by the processor 101, may be configured to store an executable program (for example, machine instructions) in an operating system or another running program, and may be further configured to store data of a user and an application, and the like.

The non-volatile memory may also store the executable program, the data of the user and the application, and the like, and may be loaded into the random access memory in advance for directly reading and writing by the processor 101.

In this embodiment of this application, the memory 102 may be configured to store a PPG signal collected by the electronic device 100.

The sensor 103 may include a plurality of sensors such as a gyroscope sensor 1031, an acceleration sensor 1032, a galvanic skin response sensor 1033, a touch sensor 1034, a bone conduction sensor 1035, and a photoelectric sensor 1036.

The gyroscope sensor 1031 may be configured to determine a moving posture of the electronic device 100. In some embodiments, angular velocities of the electronic device 100 around three axes (namely, axes x, y, and z) may be determined through the gyroscope sensor 1031.

The acceleration sensor 1032 may detect accelerations in various directions (usually on three axes) of the electronic device 100. When the electronic device 100 is still, a magnitude and a direction of gravity may be detected. The acceleration sensor 1032 may be further configured to identify a posture of the electronic device, and is used in an application such as switching between a landscape mode and a portrait mode or a pedometer.

In this embodiment of this application, the electronic device 100 may collect exercise data of the user through the gyroscope sensor 1031 and the acceleration sensor 1032, detect, based on the exercise data, whether the user is in a status of exercise, having a meal, medication, or the like, and delete, from the collected PPG signal, an abnormal PPG signal collected when the user is affected by the external factor.

The galvanic skin response sensor 1033 may be configured to measure galvanic skin response data of the user, and the data is used to reflect a change of stress and emotion of the user.

In this embodiment of this application, the electronic device 100 may collect emotional stress data of the user through the galvanic skin response sensor 1033, detect a psychological status of the user based on the emotional stress data, and delete, from the collected PPG signal, an abnormal PPG signal collected when the user is affected by the external factor.

The touch sensor 1034 is also referred to as a "touch component". The touch sensor 1034 may be disposed on the display 104, and the touch sensor 1034 and the display 104 constitute a touchscreen, which is also referred to as a "touch screen". The touch sensor 1034 is configured to detect a touch operation performed on or near the touch sensor 1034. The touch sensor may transfer the detected touch operation to the application processor to determine a type of the touch event. A visual output related to the touch operation may be provided through the display 104. In some other embodiments, the touch sensor 1034 may alternatively be disposed on a surface of the electronic device 100 at a position different from that of the display 104.

In this embodiment of this application, the electronic device 100 may detect, through the touch sensor 1034, a touch operation performed by the user on the display 104.

The bone conduction sensor 1035 may obtain a vibration signal. In some embodiments, the bone conduction sensor 1035 may obtain a vibration signal of a vibration bone of a human vocal-cord part. The bone conduction sensor 1035 may also be in contact with a body pulse to receive a blood pressure beating signal. In some embodiments, the bone conduction sensor 1035 may alternatively be disposed in a headset, to obtain a bone conduction headset. The application processor may parse heart rate information based on the blood pressure beating signal obtained by the bone conduction sensor 1035, to implement a heart rate detection function.

In this embodiment of this application, the electronic device 100 may collect a heart rate value of the user through the bone conduction sensor 1035, detect an exercise status, a psychological status, or the like of the user based on the heart rate value, and delete, from the collected PPG signal, an abnormal PPG signal collected when the user is affected by the external factor.

The photoelectric sensor 1036 is configured to monitor a cardiovascular vital sign. The photoelectric sensor 1036 includes at least one pair of light emitting diodes and a photoelectric detector. The light emitting diode serves as a light source to irradiate skin, and the photoelectric detector detects remaining transmitted or reflected light after the light is absorbed by blood and tissues in a penetration process, and converts the transmitted or reflected light into an electrical signal, to obtain a PPG signal. An intensity of the transmitted light or the reflected light changes with an arterial pulsation, and the PPG signal also follows the arterial pulsation, that is, heartbeat of the user fluctuates rhythmically. Parameters such as a heart rate, blood oxygen saturation, and blood pressure of the user may be calculated based on the PPG signal.

Blood glucose means glucose content in the blood, when the glucose content in the blood is different, an amount of light absorbed by the blood during a penetration process is different. In other words, the PPG signal has a large correlation with a blood glucose value. In this embodiment of this application, the electronic device 100 may determine a blood glucose status of the user based on the PPG signal, and determine whether the user has high blood glucose or low blood glucose, or whether the user has a blood glucose risk, or the like. For detailed descriptions of the electronic device 100 determining the blood glucose status of the user based on the PPG signal, refer to subsequent descriptions. Details are not described herein.

It may be understood that the sensor 103 may include more or fewer sensors. This is not limited in embodiments of this application.

The display 104 may be configured to display an image, a video, and the like.

In this embodiment of this application, the electronic device 100 may display, through the display 104, collection progress of the PPG signal and the blood glucose status of the user, including the quantity of occurrences of the blood glucose abnormality, the blood glucose risk, and the like of the user. For detailed content displayed on the display 104, refer to a user interface described subsequently. Details are not described herein.

The motor 105 may generate a vibration prompt. The motor 105 may be configured to provide an incoming call vibration prompt and a touch vibration feedback. For example, touch operations performed on different applications (for example, photographing and audio playback) may correspond to different vibration feedback effects.

The wireless communication module 106 may provide a wireless communication solution that is applied to the electronic device 100, and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 106 may be one or more components integrating at least one communication processor module. The wireless communication module 106 receives an electromagnetic wave through an antenna, performs demodulation and filtering processing on an electromagnetic wave signal, and sends a processed signal to the processor 101. The wireless communication module 106 may further receive a to-be-sent signal from the processor 101, perform frequency modulation and amplification on the signal, and convert the signal into an electromagnetic wave for radiation through the antenna.

In some embodiments, the antenna of the electronic device 100 is coupled to the wireless communication module 106, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (Beidou navigation satellite system, BDS), a quasi-zenith satellite system (quasi-zenith satellite system, QZSS), and/or a satellite based augmentation system (satellite based augmentation systems, SBAS).

It may be understood that the structure shown in embodiments of this application does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented as hardware, software, or a combination of software and hardware.

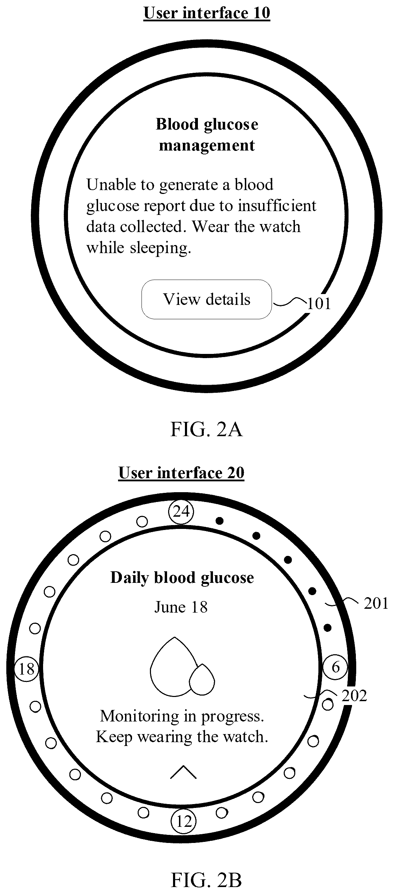

**FIG. 2A to FIG. 2E** **show example user interfaces of an electronic device 100 according to an embodiment of this application.**

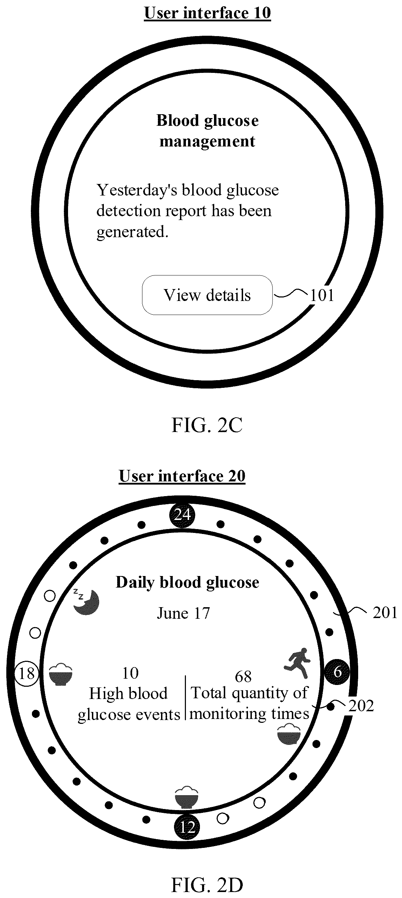

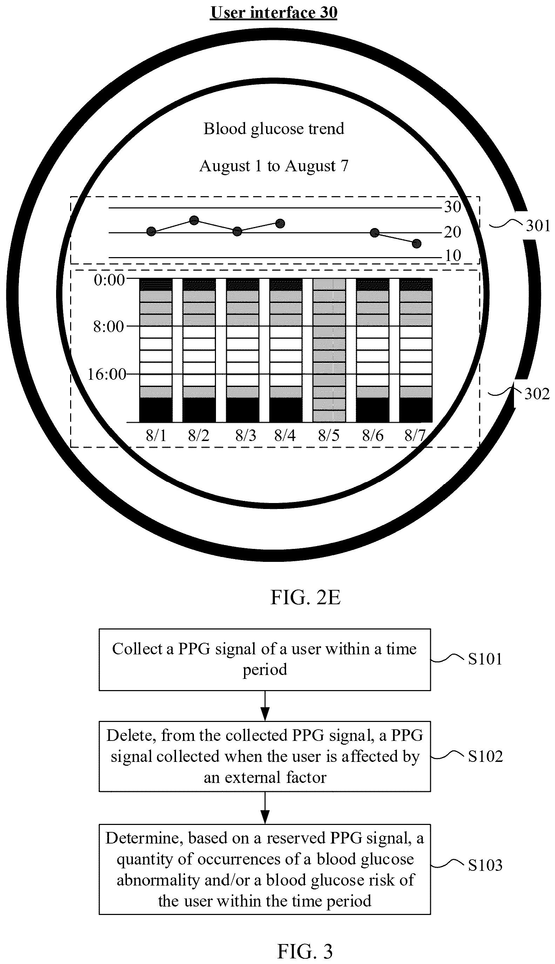

FIG. 2A and FIG. 2B show user interfaces displayed on the electronic device 100 in a process of collecting data (a PPG signal) and monitoring blood glucose. FIG. 2C and FIG. 2D show user interfaces displayed on the electronic device 100 when the electronic device 100 evaluates a blood glucose status of a user based on the collected data. FIG. 2E shows a user interface involved when the electronic device 100 presents the blood glucose status of the user.

In a process of blood glucose monitoring by the electronic device 100, if the electronic device 100 has not collected enough data, the electronic device 100 may display a user interface 10 shown in FIG. 2A. The user interface 10 displays the following prompt information: Unable to generate a blood glucose report due to insufficient data collected. Wear the watch while sleeping.

In addition, the user interface 10 further includes an option 101. When the electronic device 100 detects a user operation performed on the option 101, for example, a tap operation, the electronic device 100 may display a user interface 20 shown in FIG. 2B in response to the operation.

As shown in FIG. 2B, the user interface 20 may include two areas: a watch face area 201 and an information presentation area 202.

Content presented in the watch face area 201 indicates collection progress of the data. The watch face area 201 is presented as a ring around an edge of the user interface 20. The watch face area 201 may display 24 scales, and one scale corresponds to one hour. The 24 scales indicate 24 hours in a day. When the scale is a black dot, it indicates that the electronic device 100 has collected the PPG signal within one hour corresponding to the scale. When the scale is a white dot, it indicates that the electronic device 100 has not collected the PPG signal within one hour corresponding to the scale. It can be learned from FIG. 2B that the electronic device 100 has currently collected PPG signals from 0:00 to 5:00 on June 18.

The information presentation area 202 presents blood glucose monitoring information output by the electronic device 100. Because the electronic device 100 currently has not collected enough data, the electronic device 100 fails to evaluate the blood glucose status of the user, and the information presentation area 202 displays the following prompt information: Monitoring in progress. Keep wearing the watch.

In a process of blood glucose monitoring by the electronic device 100, if the electronic device 100 has collected enough data, the electronic device 100 may display a user interface 10 shown in FIG. 2C. The user interface 10 displays the following prompt information: Yesterday's blood glucose monitoring report has been generated.

When the electronic device 100 detects a user operation performed on the option 101, for example, a tap operation, the electronic device 100 may display a user interface 20 shown in FIG. 2D in response to the operation.

As shown in FIG. 2D, the user interface 20 presents the blood glucose status of the user evaluated by the electronic device 100.

It can be learned from the watch face area 201 shown in FIG. 2D that the electronic device 100 has collected the PPG signal from 0:00 to 9:00, 11:00 to 17:00, and 20:00 to 24:00 on June 17, and has not collected the PPG signal from 9:00 to 11:00 and 17:00 to 20:00.

In addition, an icon of an exogenous event that has occurred on June 17 is further marked around the watch face area 201. The exogenous event may include but is not limited to one or more of the following: a diet event, an exercise event, a sleep event, a wake-up event, a medication event, or the like. In addition, a display position of the icon is located near a scale corresponding to occurrence time of the exogenous event. It can be learned from FIG. 2D that the user does exercise around 6:00, has meals around 8:00, 12:00, and 18:00, and goes to sleep between 20:00 and 21:00 on June 17.

In addition, it can be learned from the information presentation area 202 shown in FIG. 2D that the electronic device 100 evaluates the blood glucose status of the user based on data collected on June 17. The blood glucose status includes a total of 68 times that the electronic device 100 monitors blood glucose of the user, in which there are 10 high blood glucose events.

It can be learned from FIG. 2A to FIG. 2D that the electronic device 100 may evaluate the blood glucose status of the user on a daily basis. The blood glucose status includes a total quantity of times that the electronic device 100 monitors blood glucose and a quantity of occurrences of high blood glucose events.

It may be understood that the electronic device 100 may evaluate the blood glucose status of the user over another duration or when another trigger condition is met. For example, the electronic device 100 may evaluate the blood glucose status of the user each time the electronic device 100 monitors whether the user has a blood glucose abnormality event. This is not limited in embodiments of this application.

In addition, the blood glucose status obtained by the electronic device 100 through evaluation is not limited to a quantity of occurrences of high blood glucose and a total quantity of monitoring times shown in FIG. 2D. For example, the blood glucose status may include a quantity of occurrences of low blood glucose, or a total quantity of occurrences of high blood glucose and low blood glucose. For another example, the blood glucose status may include a time distribution status of occurrences of the blood glucose abnormality event. For another example, the blood glucose status may include a blood glucose risk, and the blood glucose risk indicates a frequency at which a blood glucose abnormality occurs, and may be specifically a ratio of a quantity of occurrences of the blood glucose abnormality to the total quantity of monitoring times. The blood glucose status obtained by the electronic device 100 through evaluation is not limited in embodiments of this application.

FIG. 2E shows a user interface 30 involved when the electronic device 100 evaluates the blood glucose status of the user in a plurality of days based on data of the user in the plurality of days.

As shown in FIG. 2E, the user interface 30 may include a line chart 301 and a bar chart 302. The line chart 301 indicates a blood glucose risk of the user on each day from August 1 to August 7. In addition, the electronic device 100 has not collected the PPG signal of the user on August 5. Therefore, the blood glucose risk is not obtained through calculation on August 5. The bar chart 302 indicates a time period in which the electronic device 100 has collected the PPG signal and a time period in which the electronic device 100 has not collected the PPG signal each day from August 1 to August 7. A black square indicates a time period in which the electronic device 100 has collected the PPG signal at night, a white square indicates a time period in which the electronic device 100 has collected the PPG signal at daytime, and a gray square indicates a time period in which the electronic device 100 has not collected the PPG signal.

It can be learned from FIG. 2E that the user may learn, based on the line chart, a fluctuation status of the blood glucose risk of the user in several days, and may learn, based on the bar chart, a distribution status of collection time of the data of the user, to prompt the user to change a lifestyle and adjust the blood glucose of the user.

It may be understood that the user interfaces shown in FIG. 2A to FIG. 2E are merely examples for description, and do not constitute a limitation on this solution.

**FIG. 3** **is an overall method flowchart of a blood glucose monitoring method according to an embodiment of this application.**

As shown in FIG. 3, the blood glucose monitoring method may include the following steps.

**S101:** An electronic device 100 collects a PPG signal of a user within a time period.

The PPG signal may be used to reflect a blood glucose status of the user. For example, the electronic device 100 may collect the PPG signal through a photoelectric sensor. For detailed descriptions of the photoelectric sensor, refer to the descriptions of the photoelectric sensor 1036. Details are not described herein.

During specific implementation, in a process in which the user uses the electronic device 100, the electronic device 100 may continuously collect the PPG signal.

In this embodiment of this application, the time period may also be referred to as a first time period, and the PPG signal collected within the time period may also be referred to as a first PPG signal.

**S102:** The electronic device 100 deletes, from the collected PPG signal, a PPG signal collected when the user is affected by an external factor.

The external factor may include but is not limited to one or more of the following: diet, medication, exercise, psychology, or the like. A blood glucose value is abnormal when the user is affected by the external factor. The abnormality is compared with a blood glucose value when the user is not affected by the external factor. The abnormality includes but is not limited to: a high fluctuation frequency of blood glucose, high or low blood glucose, and the like.

It can be learned that the blood glucose status of the user that is evaluated by the electronic device 100 based on the PPG signal collected when the user is affected by the external factor is inaccurate. Therefore, the electronic device 100 may delete, from the collected PPG signal, the PPG signal collected when the user is affected by the external factor.

During specific implementation, in a process of collecting the PPG signal, the electronic device 100 may further determine, in any one of the following manners, whether the user is affected by the external factor:
(1) The electronic device 100 determines, through a sensor, whether the user is affected by the external factor.
   For example, the electronic device 100 may collect exercise data of the user through a gyroscope sensor, an acceleration sensor, or the like, to determine whether the user is doing exercise or having a meal; and collect emotional stress data of the user through a galvanic skin response sensor and collect a heart rate value of the user through a bone conduction sensor, to determine whether the user is in an abnormal psychological status such as excitement, anxiety, or tension.
(2) The electronic device 100 determines, based on data input by the user, whether the user is affected by the external factor.

For example, the electronic device 100 may detect a user operation that is input by the user and that indicates start of exercise, to determine that the user is currently doing exercise. For another example, the electronic device 100 may detect a schedule that is input by the user and that indicates a need to take glucose-lowering medication at 9:00 every day, to determine, based on medication time and duration of efficacy of the glucose-lowering medication, a time period in which the user is affected by the medication.

It may be understood that the electronic device 100 may further determine whether the user is affected by the external factor in another manner. This is not limited in embodiments of this application.

**S103:** The electronic device 100 determines, based on a reserved PPG signal, a quantity of occurrences of a blood glucose abnormality and/or a blood glucose risk of the user within the time period.

The electronic device 100 may trigger execution of step S103 in the following three cases.
(1) Collection duration in which the electronic device 100 collects the PPG signal reaches preset duration.
   For example, the preset duration is one hour. After collecting a PPG signal within one hour and deleting a PPG signal affected by the external factor, the electronic device 100 may evaluate the blood glucose status of the user based on a reserved PPG signal.
(2) The reserved PPG signal after deletion by the electronic device 100 includes a PPG signal within preset duration.

For example, the preset duration is one hour. After collecting and screening a PPG signal, the electronic device 100 uses a reserved PPG signal to evaluate the blood glucose status of the user when the reserved PPG signal includes the PPG signal within one hour.

It can be learned from case 1 and case 2 that the electronic device 100 is triggered to evaluate the blood glucose status of the user based on the collected PPG signal only when the electronic device 100 collects enough PPG signals.

In some implementations, in a process in which the electronic device 100 collects the PPG signal, if the electronic device 100 has not collected enough PPG signals, for example, when collection duration of the PPG signal does not reach specified duration, the electronic device 100 may output prompt information, to prompt the user to continue to use the electronic device 100 to collect the PPG signal. For example, the electronic device 100 may display a user interface shown in FIG. 2A or FIG. 2B, where text prompt information is displayed to prompt the user to continue to wear the watch for data collection. In this way, the user can be reminded to perform adjustment in a timely manner, so that the electronic device 100 collects enough PPG signals.

(3) The electronic device 100 detects a user operation of blood glucose evaluation by the user.

In this case, after detecting that the user triggers the operation of blood glucose evaluation, the electronic device 100 may evaluate the blood glucose status of the user based on a currently collected and screened PPG signal.

It may be understood that the electronic device 100 may further trigger step S103 in another case. This is not limited in embodiments of this application.

It can be learned that the electronic device 100 evaluates the blood glucose status of the user based on a reserved PPG signal after screening within a time period, which may include: determining the quantity of occurrences of the blood glucose abnormality and/or the blood glucose risk of the user within the time period. The blood glucose risk indicates a frequency at which the blood glucose abnormality occurs within the time period.

In addition, the electronic device 100 may display a blood glucose evaluation status of the user. In this way, the user can view an evaluation result of the blood glucose of the user, and perform self-management and control on the blood glucose.

During specific implementation in which the electronic device 100 determines the quantity of occurrences of the blood glucose abnormality of the user based on the reserved PPG signal, the quantity of occurrences of the blood glucose abnormality is a quantity of samples with the blood glucose abnormality in a plurality of samples, where each sample includes a reserved PPG signal within preset duration within the time period. In other words, the electronic device 100 may divide the reserved PPG signal into N (N is a positive integer, and N ≥ 1) sub-time periods by using the preset duration (for example, 15 minutes) as an interval, and separately perform blood glucose monitoring, to determine whether there is a blood glucose abnormality event within the sub-time period, and determine a quantity of blood glucose abnormality events determined within the time period as the quantity of occurrences of the blood glucose abnormality of the user within the time period. The quantity N of monitoring times of the electronic device 100 within the time period is a total quantity of monitoring times.

The blood glucose abnormality event may include a high blood glucose event and/or a low blood glucose event. The high blood glucose event indicates that the blood glucose of the user is higher than a normal range of blood glucose, and the low blood glucose event indicates that the blood glucose of the user is lower than a normal range of blood glucose.

For example, it is assumed that in a process in which the electronic device 100 collects the PPG signal within 24 hours of a previous day, the electronic device 100 performs 68 times of blood glucose monitoring based on reserved PPG signals after screening at an interval of PPG signals within 15 minutes, and there are 10 high blood glucose events. For example, the electronic device 100 may display a user interface 20 shown in FIG. 2D, and present, through the user interface 20, a blood glucose status evaluated by the electronic device 100 based on data of one day.

The electronic device 100 may determine, based on a PPG signal within a sub-time period and by using a short-term blood glucose model, whether there is a blood glucose abnormality event within the sub-time period. The short-term blood glucose model is obtained through training based on a PPG signal whose blood glucose result is known within a plurality of time periods. For details about the short-term blood glucose model and detailed descriptions of determining the blood glucose abnormality event by using the short-term blood glucose model, refer to subsequent descriptions. Details are not described herein.

During specific implementation in which the electronic device 100 determines the blood glucose risk of the user based on the reserved PPG signal, there may be the following two calculation manners:
(1) Blood glucose risk = Quantity of occurrences of blood glucose abnormality/Total quantity of monitoring times
   In this case, the blood glucose risk is a ratio of the quantity of occurrences of the blood glucose abnormality to the total quantity of monitoring times within the time period obtained by the electronic device 100 through calculation. In other words, a higher frequency at which the blood glucose abnormality event occurs indicates a higher blood glucose risk, and a lower frequency at which the blood glucose abnormality event occurs indicates a lower blood glucose risk. For detailed descriptions of the quantity of occurrences of the blood glucose abnormality and the total quantity of monitoring times, refer to related descriptions in the foregoing descriptions of the electronic device 100 calculating the quantity of occurrences of the blood glucose abnormality. Details are not described herein.
(2) A long-term blood glucose model is used to determine the blood glucose risk based on the reserved PPG signal.

The long-term blood glucose model is obtained through training based on a PPG signal whose blood glucose risk is known within a plurality of time periods. For details about the long-term blood glucose model and detailed descriptions of determining the blood glucose risk by using the long-term blood glucose model, refer to subsequent descriptions. Details are not described herein.

In this embodiment of this application, the reserved PPG signal may also be referred to as a second PPG signal.

In some embodiments, in a process in which the electronic device 100 collects the PPG signal, the electronic device 100 may display collection progress of data. The collection progress may be a time period (for example, a first time period) in which the electronic device 100 has collected the PPG signal and a time period (for example, a second time period) in which the electronic device 100 has not collected the PPG signal. In this way, the user can learn, based on the collection progress, progress of collecting the PPG signal by the electronic device 100, so that the user performs adjustment in a timely manner, and the electronic device 100 collects enough PPG signals.

Further, when the electronic device 100 is a wearable device, the collection progress may be displayed as a progress bar around an edge of a watch face. For example, the progress bar may represent 24 hours of a day. In this way, the collection progress may be cleverly combined with the watch face, so that the user can learn about collection progress of the PPG signal in a day.

Further, the electronic device 100 may further mark an occurred exogenous event and occurrence time of the exogenous event in the progress bar. The exogenous event may include but is not limited to one or more of the following: a diet event, an exercise event, a medication event, a sleep event, a wake-up event, or the like. In this way, an association between the blood glucose of the user and the exogenous event may be highlighted, so that the user, based on the exogenous event, controls a change of the blood glucose of the user by adjusting the exogenous event, thereby enhancing a self-management capability of the user on the blood glucose.

For example, for the electronic device 100 displaying the collection progress of the data, refer to the user interface 20 shown in FIG. 2B or FIG. 2D.

In some implementations, the electronic device 100 may further learn a habit of using the electronic device 100 by the user and a daily life status of the user, to predict a time period in which the user is not affected by the external factor. When the time period arrives, the electronic device 100 outputs prompt information, to remind the user to use the electronic device 100 to collect data. For example, when the electronic device 100 is a watch, the user is reminded to wear the watch in time.

In some embodiments, the electronic device 100 may further display a risk curve (for example, a first risk curve), where the risk curve may indicate a blood glucose risk determined for the user on each of M days. For example, the risk curve may be a line chart 301 in a user interface 30 shown in FIG. 2E. In this way, the user can learn about fluctuation of the blood glucose risk within several days based on the risk curve, and review and summarize the blood glucose status of the user, thereby facilitating self-management of a change of the blood glucose of the user.

Further, the electronic device 100 may further mark a reference value in the risk curve. The reference value may indicate a maximum risk value of healthy individuals, and may further indicate a warning value of the blood glucose abnormality. In other words, if a value of the blood glucose risk of the user exceeds the maximum risk value of the healthy individuals, it indicates that the blood glucose status of the user is unhealthy; and if a value of the blood glucose risk of the user exceeds the warning value of the blood glucose abnormality, it indicates that the blood glucose status of the user poses a potential danger, and the user needs to seek medical treatment in time.

The maximum risk value of the healthy individuals may be a maximum value of a blood glucose risk selected from blood glucose risks calculated based on PPG signals of healthy individuals with normal blood glucose metabolism. Similarly, the warning value of the blood glucose abnormality may be a minimum value of a blood glucose risk selected from blood glucose risks calculated based on PPG of unhealthy individuals with blood glucose abnormalities.

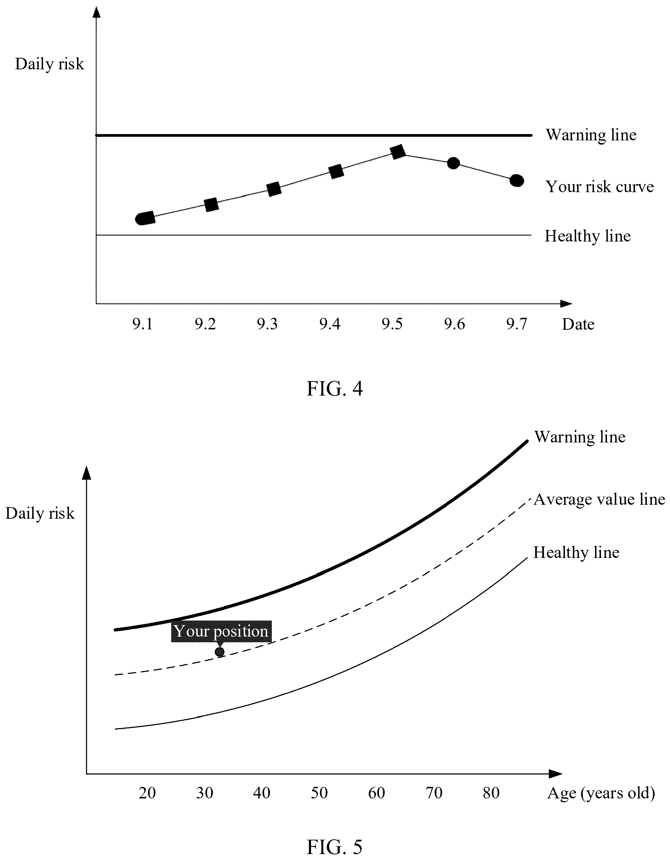

For example, FIG. 4 is a diagram of a risk curve marked with reference values according to an embodiment of this application.

It can be learned that FIG. 4 shows values of the blood glucose risk of the user on each day from September 1 to September 1. FIG. 4 further shows a healthy line and a warning line. A value of the healthy line is the maximum risk value of the healthy individuals, and a value of the warning line is the warning value of the blood glucose abnormality.

It can be learned that marking the reference value in the risk curve can help the user have a clearer understanding of a physical condition of the user, learn a blood glucose difference between the user and the healthy individuals and a blood glucose difference between the user and the unhealthy individuals, and quantify the blood glucose difference between the user and the healthy individuals, so that the user can control the blood glucose of the user in a timely manner, to avoid physical harm caused by high or low blood glucose.

In some embodiments, the electronic device 100 may further display a risk curve formed by connecting blood glucose risks of individuals of different age groups, and mark a value of a blood glucose risk of an age group of the user. The value of the blood glucose risk of the age group of the user may be determined based on the blood glucose risk of the user in a plurality of days. For example, the value of the blood glucose risk of the age group of the user is an average value of blood glucose risks of the user in the plurality of days, or is a value of the blood glucose risk of the user with a highest frequency of occurrences in the plurality of days.

For example, FIG. 5 is a diagram of risk curves of individuals of different age groups according to an embodiment of this application.

FIG. 5 shows a risk curve (a healthy line shown in FIG. 5) formed by connecting maximum values of blood glucose risks of healthy individuals of different age groups, a risk curve (a warning line shown in FIG. 5) formed by connecting minimum values of blood glucose risks of unhealthy individuals of different age groups, and an average value line obtained through calculation based on the healthy line and the warning line. For example, each point on the average value line may be an average value of risk values at a corresponding age on the healthy line and the warning line.

It can be learned from FIG. 5 that, as an age increases, there is a general trend of gradually rising blood glucose risks among individuals. The blood glucose risk of the user is between the warning line and the average value line. This indicates that the blood glucose of the user reflects that the user is unhealthy, but the body has not yet suffered significant actual harm.

It can be learned that, based on the risk curves formed by connecting the blood glucose risks of the individuals of different age groups, the user may learn a blood glucose risk status of each age group and a current blood glucose risk status of the user. This reminds the user to pay attention to physical health in a timely manner.

In some embodiments, the electronic device 100 may further display a bar chart (for example, a first bar chart). The bar chart may indicate a time period in which the PPG signal has been collected and a time period in which the PPG signal has not been collected by the user on each of the M days. For example, the bar chart may be a bar chart 302 in a user interface 30 shown in FIG. 2E. In this way, the user can view distribution of collection time of the data of the user in several days, to facilitate adjustment of a lifestyle of the user in a timely manner and more effective data collection by the device.

It can be learned from steps S101 to S103 that the electronic device 100 collects, in a non-invasive manner, the data reflects the blood glucose of the user, thereby reducing a psychological burden of the user during blood glucose monitoring. In addition, the electronic device 100 is a wearable device that can collect the data of the user in real time, and can automatically remove collected abnormal data. The user does not need to intentionally cooperate with the device for blood glucose monitoring, thereby reducing a time burden of the user. The user can perform blood glucose monitoring at any time in daily life. In addition, a volume of data collected by the device is not limited by time, and a large amount of data can be collected to evaluate the blood glucose status of the user, so that a blood glucose evaluation result is more accurate and representative. It can be learned that the blood glucose monitoring method has higher practical value in the field of non-invasive blood glucose monitoring.

**The following describes a detailed process in which an electronic device 100 identifies a blood glucose abnormality event and determines a blood glucose risk by using a blood glucose model.**

In a process of evaluating a blood glucose status of a user, the electronic device 100 may extract a feature of a PPG signal, and input the feature into a short-term blood glucose model to determine whether there is a blood glucose abnormality event.

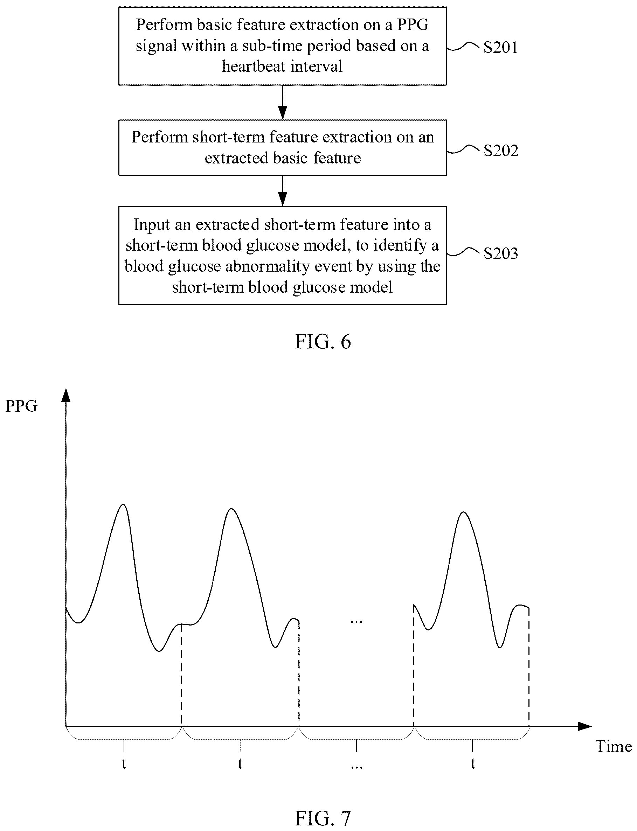

FIG. 6 is a detailed method flowchart for identifying a blood glucose abnormality event by an electronic device 100 by using a short-term blood glucose model. As shown in FIG. 6, a detailed process of identifying the blood glucose abnormality event by the electronic device 100 by using the short-term blood glucose model may include the following steps:

**S201:** The electronic device 100 performs basic feature extraction on a PPG signal within a sub-time period based on a heartbeat interval.

A continuous PPG signal within a time period may be represented as a curve that fluctuates with heartbeat of the user. FIG. 7 shows a curve drawn based on a PPG signal collected within a continuous time period. It can be learned from FIG. 7 that the curve may be divided into a plurality of groups based on a heartbeat interval t.

Specifically, the electronic device 100 may divide the PPG signal within the sub-time period into K groups based on the heartbeat interval, and extract a basic feature of each group. The PPG signal may be a PPG signal within a sub-time period divided based on preset duration in the reserved PPG signal mentioned in step S103.

The basic feature may include but is not limited to a maximum value, a minimum value, a maximum slope value, a minimum slope value, a quantity of fluctuation times, an average value, a variance, a quantile, and the like.

In other words, after the electronic device 100 performs basic feature extraction on the PPG signal, the electronic device 100 may obtain K groups of basic features, where K is a quantity of groups obtained after the PPG signal within the sub-time period is divided based on the heartbeat interval.

**S202:** The electronic device 100 performs short-term feature extraction on an extracted basic feature.

Specifically, after obtaining the K groups of basic features, the electronic device 100 may perform short-term feature extraction on a same basic feature in the K groups.

The short-term feature may include but is not limited to a maximum value, a minimum value, an average value, a variance, a quantile, and the like.

For example, it is assumed that the basic feature includes a maximum value, and the short-term feature includes an average value. After performing basic feature extraction on the PPG signal, the electronic device 100 may separately determine a maximum value in each of the K groups, to obtain K maximum values in total. When performing short-term feature extraction, the electronic device 100 may calculate an average value of the K maximum values, to determine the average value as the short-term feature obtained through extraction.

It is assumed that the basic feature includes L parameters, so that the electronic device 100 may obtain L groups of short-term features after performing short-term feature extraction on the extracted basic feature.

**S203:** The electronic device 100 inputs an extracted short-term feature into the short-term blood glucose model, to identify the blood glucose abnormality event by using the short-term blood glucose model.

The short-term blood glucose model may be used to identify the blood glucose abnormality event based on the feature of the PPG signal. The short-term blood glucose model is obtained through training based on a feature of a PPG signal whose blood glucose abnormality status is known within a plurality of time periods. An input of the short-term blood glucose model is a short-term feature, an output is a blood glucose abnormality status, and the blood glucose abnormality status indicates whether there is a blood glucose abnormality event. In this embodiment of this application, the short-term blood glucose model may also be referred to as a first blood glucose model. A PPG signal within a sub-time period may also be referred to as a first sample, and the sub-time period may also be referred to as first preset duration.

When the electronic device 100 inputs the L groups of short-term features obtained in step S202 into the short-term blood glucose model, an obtained blood glucose result may indicate whether there is a blood glucose abnormality event within the sub-time period.

Steps S201 to S203 show a process in which the electronic device 100 identifies a blood glucose abnormality event based on a PPG signal within a sub-time period obtained by dividing a time period. Similarly, the electronic device 100 may separately determine, based on a PPG signal within each sub-time period, whether there is a blood glucose abnormality event within each sub-time period, to count a quantity of occurrences of the blood glucose abnormality within the time period.

It may be understood that, when the short-term blood glucose model is trained, if a known blood glucose result of training data used includes only a high blood glucose event, and does not include a low blood glucose event, the short-term blood glucose model is used to identify only the high blood glucose event. In other words, if the short-term blood glucose model is obtained through training based on a PPG signal that is known to be a high blood glucose event within a plurality of time periods, when identifying the blood glucose abnormality event based on a feature of the PPG signal, the electronic device 100 can identify only whether there is a high blood glucose event. Similarly, if the short-term blood glucose model is obtained through training based on a PPG signal that is known to be a low blood glucose event within a plurality of time periods, when identifying the blood glucose abnormality event based on a feature of the PPG signal, the electronic device 100 can identify only whether there is a low blood glucose event.

In some implementations, the electronic device 100 may further obtain basic information of the user, screen a short-term blood glucose model suitable for the user from a plurality of short-term blood glucose models based on the basic information of the user, and identify the blood glucose abnormality event of the user by using the short-term blood glucose model.

The basic information of the user may include but is not limited to one or more of the following: a gender, an age, a medical history, a height, a weight, and the like.

In other words, the short-term blood glucose model used when the blood glucose abnormality event is identified is a personalized model determined based on the basic information of the user. This is because individuals of different genders, age groups, heights, weights, or those with different diseases have varying physiological functions and abilities to self-regulate blood glucose.

In the plurality of short-term blood glucose models, a short-term blood glucose model for a specific group of individuals is obtained through training based on training data collected from the specific group of individuals.

It can be learned that the personalized model can achieve more accurate and appropriate identification of the blood glucose of the user.

In a process of evaluating a blood glucose status of a user, the electronic device 100 may extract a feature of a PPG signal, and input the feature into a long-term blood glucose model to determine a blood glucose risk.

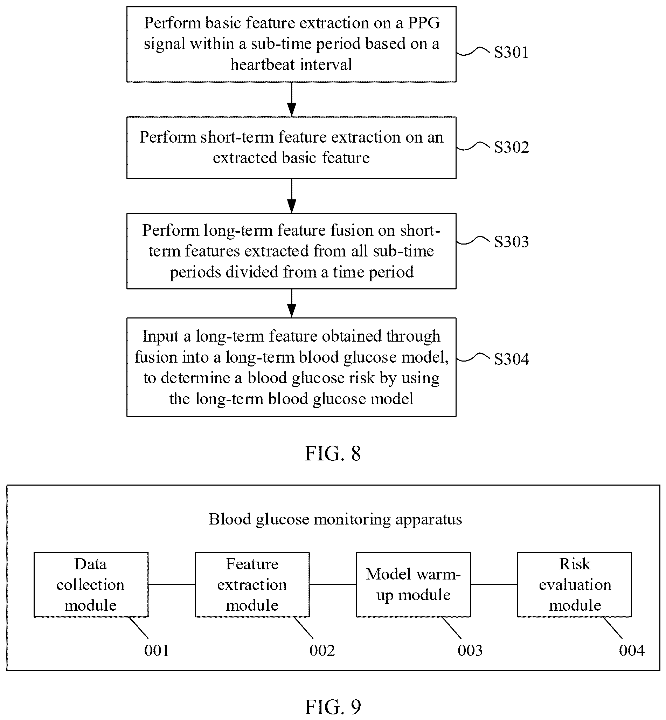

FIG. 8 shows a detailed method flowchart for determining a blood glucose risk by an electronic device 100 by using a long-term blood glucose model. As shown in FIG. 8, a detailed process in which the electronic device 100 calculates the blood glucose risk by using the long-term blood glucose model may include the following steps:
**S301:** The electronic device 100 performs basic feature extraction on a PPG signal within a sub-time period based on a heartbeat interval.
**S302:** The electronic device 100 performs short-term feature extraction on an extracted basic feature.

Descriptions of steps S301 and S302 are similar to descriptions of the foregoing steps S201 and S202, and reference may be made correspondingly. Details are not described herein.

**S303:** The electronic device 100 performs long-term feature fusion on short-term features extracted from all sub-time periods obtained by dividing a time period.

It is mentioned above in determining the quantity of occurrences of the blood glucose abnormality that the electronic device 100 divides a reserved PPG signal within a time period into a plurality of sub-time periods at an interval of preset duration, and separately determines whether there is a blood glucose abnormality event within each sub-time period, to count a quantity of occurrences of the blood glucose abnormality.

When determining the blood glucose risk, the electronic device 100 may perform the basic feature extraction and the short-term feature extraction mentioned in the foregoing steps S301 and S302 on the PPG signal within each sub-time period obtained by dividing the time period, to obtain L groups of short-term features within N sub-time periods.

For example, the long-term feature fusion may include the following two manners:
(1) Concatenating fusion

Concatenating fusion means combining same parameters to form a group of parameters with an increased quantity of parameters.

It is assumed that the short-term feature includes M parameters, and the L groups of short-term features within the N sub-time periods include N groups of L*M parameters in total. Concatenation fusion is performed on the N groups of L*M parameters, and N same parameters may be concatenated into one group of parameters, to obtain L*M long-term features, where one long-term feature includes a same parameter from each of the N groups.

### (2) Average value fusion

Average value fusion means to calculate an average value of same parameters.

It is assumed that the short-term feature includes M parameters, and the L groups of short-term features within the N sub-time periods include N groups of L*M parameters in total. Performing the average value fusion on the N groups of L*M parameters means that a value of each of the L*M parameters is extracted from each of the N groups to calculate an average value of the parameter. In this way, the average values of the L*M parameters form L*M long-term features.

It may be understood that the long-term feature fusion may further include another manner. This is not limited in embodiments of this application.

**S304:** The electronic device 100 inputs a long-term feature obtained through fusion into the long-term blood glucose model, to determine the blood glucose risk by using the long-term blood glucose model.

The long-term blood glucose model may be used to determine the blood glucose risk based on the feature of the PPG signal. The short-term blood glucose model is obtained through training based on a feature of a PPG signal whose blood glucose risk is known within a plurality of time periods. An input of the long-term blood glucose model is a long-term feature, and an output is a value of the blood glucose risk. In this embodiment of this application, the long-term blood glucose model may also be referred to as a second blood glucose model.

When the electronic device 100 inputs the L*M long-term features obtained in step S303 into the long-term blood glucose model, the output of the long-term blood glucose model is the value of the blood glucose risk.

It should be noted that when the electronic device 100 determines a blood glucose risk of the user within a time period by using the long-term blood glucose model, there can be two usage manners:
(1) The long-term blood glucose model is directly used to determine the blood glucose risk of the user within the time period.

In this case, the electronic device 100 may extract a feature directly based on a PPG signal collected by the user within the time period, and input the feature into the long-term blood glucose model, to determine the blood glucose risk of the user within the time period.

For example, it is assumed that the time period is 24 hours in a day. The electronic device 100 may directly use the long-term blood glucose model to evaluate the blood glucose risk of the user in the day.

(2) The time period is divided into a plurality of time periods, blood glucose risks in different time periods are calculated by using the long-term blood glucose model, and then the blood glucose risk within the time period is calculated through weighted summation.

In this case, the electronic device 100 needs to separately extract features based on PPG signals collected by the user within different time periods, and separately input the features into the long-term blood glucose model, to determine a blood glucose risk within each time period, thereby obtaining the blood glucose risk of the user within the time period.

For example, it is assumed that the time period is 24 hours in a day, and the plurality of time periods may include two time periods: daytime and night. The electronic device 100 may separately determine blood glucose risks during daytime and at night by using the long-term blood glucose model, and obtain the blood glucose risk within the day by performing weighted summation on the blood glucose risks during daytime and at night.

In this way, blood glucose risk evaluation can be achieved at a finer granularity based on time, so that the blood glucose risk evaluation is more accurate.

In some implementations, the electronic device 100 may further obtain basic information of the user, screen a long-term blood glucose model suitable for the user from a plurality of long-term blood glucose models based on the basic information of the user, and identify the blood glucose abnormality event of the user by using the long-term blood glucose model.

The basic information of the user may include but is not limited to one or more of the following: a gender, an age, a medical history, a height, a weight, and the like. In other words, the long-term blood glucose model used when the blood glucose risk is determined is a personalized model determined based on the basic information of the user. This is because individuals of different genders, age groups, heights, weights, or those with different diseases have varying physiological functions and abilities to self-regulate blood glucose.

In the plurality of long-term blood glucose models, a long-term blood glucose model for a specific group of individuals is obtained through training based on training data collected from the specific group of individuals.

It can be learned that the personalized model can achieve more accurate and appropriate calculation of the blood glucose risk.

In addition, it should be noted that, in a process in which the electronic device 100 performs steps S201 and S202 and steps S301 to S303, when sequentially performing basic feature extraction, short-term feature extraction, and long-term feature extraction, the electronic device 100 may delete previously used data after one of the features is extracted. This reduces a waste of storage space and improves utilization of the storage space.

**FIG. 9** **is a diagram of a structure of a blood glucose monitoring apparatus according to an embodiment of this application.**

As shown in FIG. 9, the blood glucose monitoring apparatus may include a data collection module 001, a feature extraction module 002, a model warm-up module 003, and a risk evaluation module 004.

The data collection module 001 may be configured to collect a PPG signal. Further, the data collection module may be further configured to collect external data such as basic information of a user, exercise data, emotional stress data, a heart rate, and data input by the user. The external data is used to determine whether the user is affected by an external factor. In addition, the data collection module may be further configured to screen the collected PPG signal, and delete a PPG signal collected when the user is affected by the external factor.

The feature extraction module 002 may be configured to perform feature extraction on a reserved PPG signal after deletion by the data collection module, where the feature extraction may include: basic feature extraction, short-term feature extraction, and long-term feature extraction.

The model warm-up module 003 may be configured to select, based on the basic information of the user, a personalized model suitable for the user, where the model may include a short-term blood glucose model and/or a long-term blood glucose model.

The risk evaluation module 004 may be configured to identify a blood glucose abnormality event by using the short-term blood glucose model, or determine a blood glucose risk by using the long-term blood glucose model, based on a feature extracted from the PPG signal. In addition, the risk evaluation module may be further configured to present a blood glucose status of the user, including presenting a quantity of occurrences of a blood glucose abnormality, a total quantity of monitoring times, the blood glucose risk, and the like of the user.

It may be understood that the blood glucose monitoring apparatus may be an electronic device 100. The electronic device 100 may implement a function of the data collection module through a sensor, implement a process of evaluating a blood glucose status in the data extraction module, the model warm-up module, and the risk evaluation module through a processor, and implement a function of presenting the blood glucose status of the user in the risk evaluation module through a display. In addition, the blood glucose monitoring apparatus may further include more or fewer modules. This is not limited in embodiments of this application.

It should be understood that the steps in the foregoing method embodiments may be completed by using an integrated logic circuit of hardware in the processor or instructions in a form of software. The steps in the method disclosed with reference to embodiments of this application may be directly performed and completed by a hardware processor, or may be performed and completed by a combination of hardware in the processor and a software module.

This application further provides an electronic device. The electronic device may include a memory and a processor. The memory may be configured to store a computer program. The processor may be configured to invoke the computer program in the memory, so that the electronic device performs the method performed by the electronic device 100 in any one of the foregoing embodiments.

This application further provides a chip system. The chip system includes at least one processor, configured to implement functions in the method performed by the electronic device 100 in any one of the foregoing embodiments.

In a possible design, the chip system further includes a memory, the memory is configured to store program instructions and data, and the memory is located inside or outside the processor.

The chip system may include a chip, or may include a chip and another discrete component.

Optionally, there may be one or more processors in the chip system. The processor may be implemented by using hardware, or may be implemented by using software. When the processor is implemented by using the hardware, the processor may be a logic circuit, an integrated circuit, or the like. When the processor is implemented by using the software, the processor may be a general-purpose processor, and is implemented by reading software code stored in the memory.

Optionally, there may also be one or more memories in the chip system. The memory may be integrated with the processor, or may be disposed separately from the processor. This is not limited in embodiments of this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in embodiments of this application.

For example, the chip system may be a field programmable gate array (field programmable gate array, FPGA), an application specific integrated circuit (application specific integrated circuit, ASIC), a system-on-chip (system-on-chip, SoC), a central processing unit (central processing unit, CPU), a network processor (network processor, NP), a digital signal processor (digital signal processor, DSP), a micro controller unit (micro controller unit, MCU), a programmable logic device (programmable logic device, PLD), or another integrated chip.

This application further provides a computer program product. The computer program product includes a computer program (which may also be referred to as code or instructions). When the computer program is run, a computer is enabled to perform the method performed by the electronic device 100 in any one of the foregoing embodiments.

This application further provides a computer-readable storage medium. The computer-readable storage medium stores a computer program (which may also be referred to as code or instructions). When the computer program is run, a computer is enabled to perform the method performed by the electronic device 100 in any one of the foregoing embodiments.

It should be noted that the processor in embodiments of this application may be an integrated circuit chip, and has a signal processing capability. In an implementation process, the steps in the foregoing method embodiments may be completed by using an integrated logic circuit of hardware in the processor or instructions in a form of software. The foregoing processor may be a general-purpose processor, a digital signal processor (digital signal processor, DSP), an application-specific integrated circuit (application-specific integrated circuit, ASIC), a field programmable gate array (field programmable gate array, FPGA) or another programmable logic device, a discrete gate or a transistor logic device, or a discrete hardware component. It may implement or perform the methods, the steps, and logical block diagrams that are disclosed in embodiments of this application. The general-purpose processor may be a microprocessor, or the processor may be any conventional processor or the like. The steps in the method disclosed with reference to embodiments of this application may be directly performed and completed by a hardware decoding processor, or may be performed and completed by a combination of hardware in the decoding processor and a software module. A software module may be located in a mature storage medium in the art, such as a random access memory, a flash memory, a read-only memory, a programmable read-only memory, an electrically erasable programmable memory, or a register. The storage medium is located in the memory, and a processor reads information in the memory and completes the steps in the foregoing methods in combination with hardware of the processor.

In addition, an embodiment of this application further provides an apparatus. The apparatus may be specifically a component or a module, and the apparatus may include one or more processors and memories that are connected to each other. The memory is configured to store a computer program. When the computer program is executed by one or more processors, the apparatus is enabled to perform the methods in the foregoing method embodiments.

The apparatus, the computer-readable storage medium, the computer program product, or the chip provided in embodiments of this application is configured to perform the corresponding method provided above. Therefore, for beneficial effect that can be achieved, refer to beneficial effect in the corresponding method provided above, and details are not described herein again.

The implementations of this application may be randomly combined to achieve different technical effect.

All or some of the foregoing embodiments may be implemented by using software, hardware, firmware, or any combination thereof. When software is used to implement the embodiments, all or a part of the embodiments may be implemented in a form of a computer program product. The computer program product includes one or more computer instructions. When the computer program instructions are loaded and executed on the computer, the procedure or functions according to this application are all or partially generated. The computer may be a general-purpose computer, a dedicated computer, a computer network, or other programmable apparatuses. The computer instructions may be stored in a computer-readable storage medium, or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive (solid state disk, SSD)), or the like.

A person of ordinary skill in the art may understand that all or some of the processes of the methods in embodiments may be implemented by a computer program instructing related hardware. The program may be stored in a computer-readable storage medium. When the program runs, the processes of the methods in embodiments are performed. The foregoing storage medium includes any medium that can store program code, such as a ROM, a random access memory RAM, a magnetic disk, or an optical disc.

In conclusion, the foregoing descriptions are merely embodiments of the technical solutions of the present invention, but are not intended to limit the protection scope of the present invention. Any modification, equivalent replacement, or improvement made according to the disclosure of the present invention shall fall within the protection scope of the present invention.

## Claims

1. A blood glucose monitoring method, wherein the method is applied to a wearable electronic device, and the method comprises:
collecting, by the electronic device, a first photoplethysmogram PPG signal of a user within a first time period; and
determining, by the electronic device based on a second PPG signal in the first PPG signal, a quantity of occurrences of a blood glucose abnormality and/or a blood glucose risk of the user within the first time period, wherein the blood glucose risk indicates a frequency at which the blood glucose abnormality occurs within the first time period, the second PPG signal does not comprise a PPG signal collected when the user is affected by an external factor, and the external factor comprises one or more of the following: diet, medication, exercise, or psychology.

2. The method according to claim 1, wherein the method further comprises:
displaying, by the electronic device, the quantity of occurrences of the blood glucose abnormality and/or the blood glucose risk of the user within the first time period.

3. The method according to claim 1 or 2, wherein the method further comprises:
displaying, by the electronic device, collection progress of data, wherein the collection progress indicates that the user has collected data within the first time period and has not collected data within a second time period, and the second time period is different from the first time period.

4. The method according to claim 3, wherein the collection progress is displayed as a progress bar around an edge of a watch face, and the progress bar represents 24 hours of a day.

5. The method according to claim 4, wherein an occurred exogenous event and occurrence time of the exogenous event are further marked in the progress bar, and the exogenous event comprises one or more of the following: diet, medication, exercise, sleep, or wake-up.

6. The method according to any one of claims 1 to 5, wherein the quantity of occurrences of the blood glucose abnormality within the first time period is a quantity of samples with the blood glucose abnormality in a plurality of samples, and one of the samples comprises a second PPG signal within preset duration within the first time period.

7. The method according to claim 6, wherein the blood glucose risk is equal to a ratio of the quantity of occurrences of the blood glucose abnormality within the first time period to a quantity of the plurality of samples.

8. The method according to claim 6 or 7, wherein the plurality of samples comprise a first sample, whether there is a blood glucose abnormality within first preset duration in which the first sample is located is identified by using a first blood glucose model based on a feature of the first sample, wherein the first blood glucose model is obtained through training based on a feature of a PPG signal whose blood glucose abnormality is known within a plurality of time periods.

9. The method according to claim 8, wherein the method further comprises:
obtaining, by the electronic device, basic information of the user, wherein
the first blood glucose model is obtained through screening and matching from a plurality of blood glucose models based on the basic information.

10. The method according to any one of claims 1 to 9, wherein determining, by the electronic device based on the second PPG signal in the first PPG signal, the blood glucose risk of the user within the first time period specifically comprises:
extracting, by the electronic device, a feature of the second PPG signal in the first PPG signal; and
determining, by the electronic device, the blood glucose risk within the first time period by using a second blood glucose model based on the feature of the second PPG signal, wherein the second blood glucose model is obtained through training based on a feature of a PPG signal whose blood glucose risk is known within a plurality of time periods.

11. The method according to any one of claims 1 to 10, wherein in a process in which the electronic device collects the first PPG signal of the user within the first time period, the method further comprises:
when duration in which the electronic device collects a PPG signal does not reach specified duration, outputting, by the electronic device, prompt information, to prompt the user to continue to wear the electronic device to collect the PPG signal.

12. The method according to any one of claims 1 to 11, wherein the method further comprises:
displaying, by the electronic device, a first risk curve, wherein the first risk curve indicates the blood glucose risk determined on each of a plurality of days.

13. The method according to any one of claims 1 to 12, wherein the method further comprises:
displaying, by the electronic device, a first bar chart, wherein the first bar chart indicates a time period in which a PPG signal has been collected and a time period in which a PPG signal has not been collected on each of the plurality of days.

14. An electronic device, comprising a memory, one or more processors, and one or more programs, wherein when the one or more processors execute the one or more programs, the electronic device is enabled to implement the method according to any one of claims 1 to 13.

15. A computer-readable storage medium comprising instructions, wherein when the instructions are run on an electronic device, the electronic device is enabled to perform the method according to any one of claims 1 to 13.

16. A computer program product, wherein when the computer program product runs on a computer, the computer is enabled to perform the method according to any one of claims 1 to 13.
